# EUROPEAN PATENT APPLICATION

(11) **EP 1 512 364 A1**
(43) Date of publication of application: **09.03.2005**
(21) Application number: 04020631.0
(22) Date of filing: 31.08.2004
(51) Int. Cl.: A61B 1/005

(54) **Endoscope**

(30) Priority: 03.09.2003 JP 2003311470
(71) Applicant: Olympus Corporation, Shibuya-ku, Tokyo (JP)
(72) Inventor: Matsumoto, Jun, Hachioji-shi Tokyo (JP); Nihei, Toshiyuki, Hachioji-shi Tokyo (JP); Kagawa, Ichiro, Hachioji-shi Tokyo (JP)
(74) Representative: von Hellfeld, Axel, Dr. Dipl.-Phys.

(57) **Abstract**

An endoscope comprising an operation part (1), and an intracavital insertion part (2) attached to the operation part (1) and formed of a flexible tube (10), characterized in that the yarn employed for the component member of the flexible tube (10) and the yarn employed for fixing the internal member contained in the flexible tube (10) respectively contain aramid fiber.

## Description

The present invention relates to an endoscope, and in particular, to an endoscope which is improved in a flexible tube constituting an insertion part thereof, in the fixing of an optical fiber bundle assembly which is contained in the insertion part, and in the fixing of channels.

The insertion part of an endoscope which is adapted to be introduced into a body cavity is constituted of a flexible tube 10 in which an optical fiber bundle, such as an image guide and various kinds of channels, are accommodated.

This flexible tube constituting the insertion part of the endoscope is generally constructed such that it comprises a helical tube formed of a strip member which is helically wound so as to provide a tubular structure having a constant diameter, a net tube covering the helical tube and formed of yarn which is knitted so as to form a tubular structure, and an outer skin member covering the outer peripheral surface of the net tube. As for the raw materials of the helical tube, it is possible to employ a stainless steel strip or a phosphor bronze strip. As for the raw materials of the net tube, it is possible to employ a stainless steel wire, a carbon fiber, etc. (see for example, JP Laid-open Patent Publication (Kokai) No. 10-24012 (1998)).

However, since the stainless steel strip and the phosphor bronze strip are relatively large in specific gravity and hence relatively heavy, an intermediate portion of the flexible tube, e.g. an intermediate portion located between the proximal portion of the flexible tube and the distal end portion of the flexible tube which is inserted into a living body, is caused to sag down during the operation of the endoscope, generating those portions of the flexible tube which are not straight line or arc, thus raising the problem that the operability at the time of inserting the endoscope is deteriorated. Further, since the stainless steel wire to be employed as a raw material for the net tube is also relatively large in specific gravity and hence relatively heavy, there is also a problem that the operability at the time of inserting the endoscope is deteriorated.

On the other hand, the optical fiber bundle, such as a light guide and an image guide to be accommodated in the insertion part of the endoscope, is inserted into a protective member so as to be protected from discontinuity. Conventionally, the protective member is constructed such that it is formed of protective tube made of porous fluorine resin or rubber, and the outer peripheral surface of both end portions of the protective tube is wound by silk thread or nylon thread so as to fix both end portions of the optical fiber bundle (see for example, JP Laid-open Patent Publication (Kokai) No. 5-323210 (1993) and JP Laid-open Patent Publication (Kokai) No. 2001-61774).

Further, various kinds of channels to be accommodated inside the insertion part of the endoscope, such as a treatment member channel, an air supply channel and a water supply channel, are assembled in such a manner that the distal end portion of the channel tube is secured to the outer peripheral wall of a channel pipe by winding a silk or nylon thread around the outer peripheral wall of the channel tube thereby securing the distal end of these channel tube to the channel pipe (see for example, JP Laid-open Patent Publication (Kokai) No. 5-323210 (1993) and JP Laid-open Patent Publication (Kokai) No. 2001-61774).

On the other hand, the disinfection or sterilization treatment of the medical endoscope has been conventionally performed by mainly making use of a disinfecting gas such as ethylene oxide gas or a disinfecting liquid. However, in view of the problems with regard to safety, public nuisance and running cost involved in the conventional disinfection or sterilization treatment, there is an increasing trend to employ an autoclave sterilization (high-pressure/high-temperature steam sterilization) wherein the waste liquid produced therefrom is water which is high in safety and which is pollution-free and cheap in running cost.

However, the employment of such an autoclave sterilization is accompanied with various problems associated with the aforementioned flexible tube, optical fiber bundle and the channel tube.

Namely, when the net tube to be used for the flexible tube is formed by knitting a common stainless steel wire, water vapor used during the autoclave sterilization (high-pressure/high-temperature steam sterilization) can enter and remain in the interstices of the net-like wire structure, thereby giving rise to the generation of rust and hence the generation of discontinuity of the wire in the net-like wire structure (see for example, JP Laid-open Patent Publication (Kokai) No. 2001-46330). Further, when a net tube made of carbon fiber is employed, the carbon fiber may be caused to break on the occasion of knitting the carbon fiber, and therefore, there are many reasons why it may become difficult to form a net tube having a sufficient mechanical strength.

Furthermore, the silk or nylon thread that has been wound around the outer peripheral surface of the protective tube for securing the optical fiber bundle is caused to become free of the outer peripheral surface of the protective tube due to the hydrolysis of the fibrous polymer by the effects of the water vapor molecule used in the autoclave sterilization (high-pressure/high-temperature steam sterilization). As a result, the opposite ends of the optical fiber bundle are caused to become free of the protective tube and hence caused to break, thereby possibly giving rise to the problem that it may become impossible to obtain satisfactory illumination and images.

Likewise, the silk or nylon thread that has been wound around the outer peripheral surface of the channel tube for securing the channel tube to the channel pipe is caused to become free of the outer peripheral surface of the channel tube due to the hydrolysis of the fibrous polymer by the effects of the water vapor used in an autoclave sterilization, thereby possibly giving rise to the problem that it may become impossible to obtain the predetermined performance of the channel tube.

A main object of the present invention is to provide an endoscope which is capable of overcoming the aforementioned problems and is free from the aforementioned problems even if the endoscope is subjected to autoclave sterilization (high-pressure/high-temperature steam sterilization).

According to a first aspect of the present invention, there is provided an endoscope comprising an operation part, and an intracavital insertion part attached to the operation part and formed of a flexible tube, wherein the flexible tube comprises a component member formed of yarn containing aramid fiber.

According to a second aspect of the present invention, there is provided an endoscope comprising an operation part, and an intracavital insertion part attached to the operation part and formed of a flexible tube, wherein an internal member housed in the flexible tube is secured to the flexible tube by means of yarn containing aramid fiber.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a side view illustrating the entire structure of an endoscope according to one embodiment of the present invention;
FIG. 2 is a cross-sectional view illustrating partially a flexible tube of an endoscope according to one embodiment of the present invention;
FIG. 3 is a side view illustrating one example of an image guide assembly housed in the flexible tube of an endoscope according to one embodiment of the present invention; and
FIG. 4 is a cross-sectional view illustrating a secured portion formed between a channel pipe and a channel tube housed in the flexible tube of an endoscope according to one embodiment of the present invention.

The endoscope according to one embodiment of the present invention is featured in that the yarn forming the component member of the flexible tube or the yarn employed for fixing the internal member housed in the flexible tube is formed of aramid fiber.

In the endoscope according to one embodiment of the present invention, the flexible tube is generally constructed such that it comprises a helical tube formed by helically winding a strip member, a net tube covering the helical tube, and an outer skin member covering the outer peripheral surface of the net tube, wherein the component member of the flexible tube is a net tube formed by knitting yarn made of aramid fiber.

When the net tube is formed by making use of yarn made of aramid fiber, the net tube can be made light in weight, thereby making the endoscope excellent in operability. Further, in this case, even if the endoscope is subjected to autoclave sterilization, the yarn constituting the net tube can be prevented from getting rusty or being disconnected. As a result, it is now possible to obtain an endoscope which is provided with a flexible tube which is free from being deteriorated in mechanical strength or flexibility even if the endoscope is subjected to autoclave sterilization.

Incidentally, it is preferable that the net tube is secured, through adhesion, to the outer skin member. By doing so, it is possible to further improve the mechanical strength of the flexible tube.

Further, the internal member housed inside the flexible tube is an optical fiber bundle comprising a large number of optical fibers which are bundled en bloc. This optical fiber bundle is introduced into a thin tube, thus covering the outer peripheral surface of the optical fiber bundle with the thin tube. Further, the end portion of the optical fiber bundle thus introduced is fixed by winding yarn made of aramid fiber around this end portion of the optical fiber bundle.

Since this end portion of the optical fiber bundle is fixed by winding yarn made of aramid fiber around it as described above, even if the endoscope is subjected to autoclave sterilization, there is little possibility of the yarn disintegrating and the optical fiber bundle breaking up.

Incidentally, it is preferable for the fixed portion, where the yarn is wound around, to be coated with an adhesive. By doing so, the fixing of the optical fiber bundle can be further reinforced.

The internal member housed inside the flexible tube is at least one channel tube, wherein one end of this channel tube is externally covered on the channel pipe attached to a distal end portion of the intracavital insertion part. Then, yarn made of aramid fiber is wound around the externally covered portion of the channel tube overlapping the channel pipe, thereby fixing it to the channel pipe.

Since the externally covered portion of the channel tube over the channel pipe is fixed by winding yarn made of aramid fiber around the externally covered portion of the channel tube overlapping the channel pipe as described above, even if the endoscope is subjected to autoclave sterilization, there is little possibility of the yarn disintegrating and the channel tube being disengaged from the channel pipe.

Incidentally, it is preferable that the fixed portion where the yarn is wound around be coated with an adhesive. By doing so, the fixing of the channel tube can be further reinforced.

It is preferable in the endoscope according to one embodiment of the present invention for the aramid fiber constituting the yarn to be copolyparaphenylene-3,4'-oxydiphenylene-terephthalamide, which can be obtained by copolymerizing terephthalic chloride and paraphenylene diamine, using diamine having ether linkage as a third component. It is more preferable that 50 mol% of the aforementioned diamine be constituted of 3,4'-diaminodiphenyl ether, and the aforementioned fiber be a high-orientation fiber that can be obtained by dry/wet spinning copolyparaphenylene-3,4'-oxydiphenylene-terephthalamide, and then by subjecting the resultant fiber to ultra-orientation.

Since the aramid fiber obtained in this manner is excellent in mechanical characteristics, fatigue resistance, heat characteristics, dimension stability, chemical resistance and wet heat resistance, the aramid fiber can be suitably employed in the present invention.

According to one embodiment of the present invention, even if the endoscope is subjected to autoclave sterilization, the yarn can be prevented from rusting and being fractured or disintegrated. As a result, it is possible to provide an endoscope which is excellent in manipulability and in resistance to sterilization treatment, in that the flexible tube therein can be prevented from being damaged and the internal member housed inside the flexible tube as well as at the fixing portion thereof can be prevented from being damaged.

Next, preferable embodiments in carrying out the present invention will be explained.

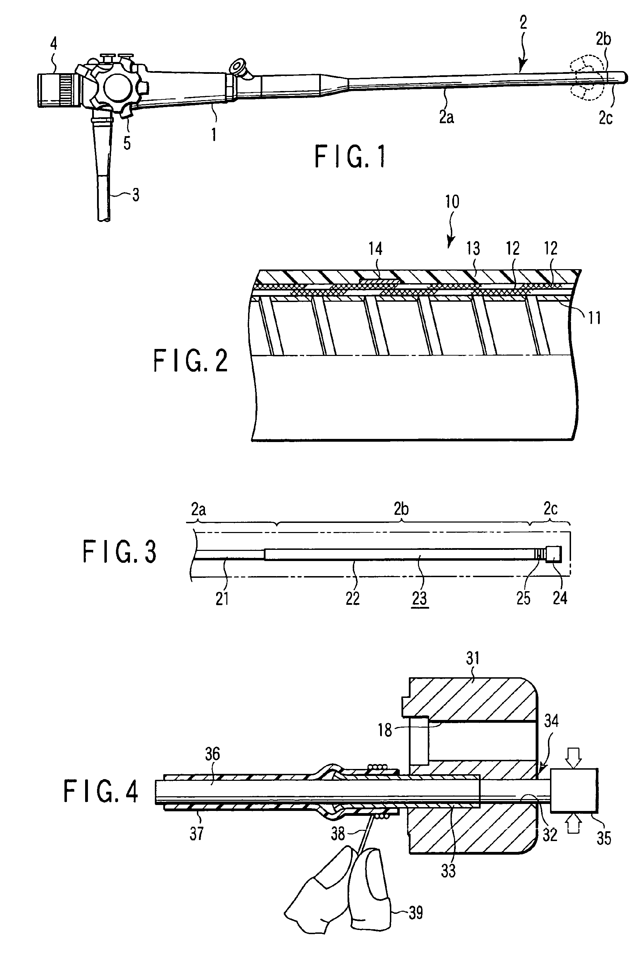

FIG. 1 illustrates the entire structure of an endoscope according to one embodiment of the present invention. Referring to FIG. 1, reference numeral 1 denotes an operation part, 2 an insertion part, 3 a universal cord, and 4 an eyepiece part. The insertion part 2 is constructed such that most of the length thereof extending from its proximal end portion formed contiguous to the operation part to the distal end portion thereof is constituted of a flexible portion 2a to which are successively attached an angle portion 2b which is enabled to flex through the remote manipulation of the operation part and a rigid distal end portion 2c having an objective optical system, etc. accommodated therein.

The insertion part 2 is formed of a flexible tube. Inside the flexible tube constituting the insertion part 2, there are inserted, as well known, not only an optical fiber bundle such as a light guide for transmitting illumination light and an image guide for transmitting an optical image of the object to be observed, but also channel tubes constituting various kinds of channels such as a treating tool (forceps, etc.) inserting channel, an air supply channel and a water supply channel.

Further, an angle manipulating wire is coupled to the distal end portion of the angle portion 2b or to the proximal end portion of rigid distal end portion 2c. The operation part 1 is provided with an angle manipulation knob 5, so that the angle manipulating wire is enabled to be pushed or pulled through the manipulation of the angle manipulation knob 5, thereby enabling the angle portion 2b to be curved. Incidentally, reference numeral 4 denotes an eyepiece part.

The endoscope according to one embodiment of the present invention is characterized in that the yarn to be employed in the endoscope as explained above is formed by making use of aramid fiber. Namely, in a first aspect of the present invention, the net tube, which is a part of the flexible tube constituting the insertion part 2 of the endoscope, is formed of yarn made of aramid fiber. Further, in a second aspect of the present invention, yarn made of aramid fiber is employed for fixing the optical fiber bundle. Furthermore, in a third aspect of the present invention, yarn made of aramid fiber is employed for fixing the channel tube.

The aramid fiber is another name of aromatic polyamide fiber and is distinguished from aliphatic polyamide fiber. This aramid fiber can be classified into two kinds, i.e. meta type and para type. Since the para type aramid fiber is higher in mechanical strength as compared with the meta type aramid fiber, the para Lype aramid fiber is more suited for use in the present invention.

As for specific examples of the para type aramid fiber, there are known polyparaphenylene terephthalamide (PPTA, tradename: Kepler, Du Pont Co., Ltd.) and copolyparaphenylene-3,4'-oxydiphenylene terephthalamide (tradename: Technora, Teijin Co., Ltd.). Among them, copolyparaphenylene-3,4'-oxydiphenylene terephthalamide (tradename: Technora, Teijin Co., Ltd.) is especially suited for use in the present invention, since this compound is almost free from deterioration in mechanical strength even if this compound is subjected to autoclave sterilization. Since Kepler is formed through wet spinning in a 100% solution of sulfuric acid, the resultant fiber is caused to have a finely projected/recessed surface which is easily attacked by water. Therefore, Kepler is inferior in wet/heat resistance to Technora.

Technora is a kind of fiber which is constituted of copolyparaphenylene-3,4'-oxydiphenylene-terephthalamide, which can be obtained by copolymerizing terephthalic chloride and paraphenylene diamine using diamine having ether linkage as a third component. As for specific examples of copolyparaphenylene-3,4'-oxydiphenylene-terephthalamide, it is preferable to employ those which can be obtained by making use of a diamine component wherein 50 mole% thereof is constituted of 3,4-diaminodiphenyl ether. Technora can be obtained by dry/wet spinning aforementioned copolymer, the resultant thread being subsequently subjected to ultra-orientation to thereby obtain a high-orientation fiber.

Next, remarkable features of this Technora which can be most suitably employed in the present invention will be explained.

Technora is excellent in mechanical characteristics, fatigue resistance, heat characteristics, dimension stability, chemical resistance and wet heat resistance. Especially, Technora is excellent in wet heat resistance, so that even if Technora is exposed to a saturated water vapor of 120°C for a long period of time (400 hours), the strength retention rate would be 100%, indicating substantially no deterioration. Further, if Technora is to be exposed to a saturated water vapor of 100 to 200°C for a period of 100 hours, the strength retention rate of Technora is not substantially deteriorated as long as the temperature is limited to 150°C or less. Thus, if the retention temperature exceeds over 150°C, Technora is caused to initiate the deterioration thereof. Whereas, when polyester fiber is exposed to a saturated water vapor of 120°C, the polyester fiber is immediately caused to deteriorate, thus rendering the strength retention rate thereof to deteriorate down to 20% within 400 hours of exposure.

The present inventors have conducted the following experiments in order to determine the shrinkage percentage and tensile strength of five kinds of threads, each containing Technora. Specifically, a weight 50g in mass was attached to each of the threads each having an initial length of 1m and kept suspended from the thread. Then, under this condition, each of the threads was exposed to an atmosphere of 135°C and 2 atm. for 5 minutes. This exposing operation was repeated 600 times, after which the length of each of the threads was measured to determine the shrinkage percentage. Further, the initial tensile strength of each of the threads and the final tensile strength thereof after 600 times of the repetition of the aforementioned exposing operation were measured. The results are shown in the following Table 1.

**Table 1**

| Kinds of thread | Shrinkage (%) | Tensile Initial | strength Aft.expos. | Thread diam. (mm) |
|---|---|---|---|---|
| Silk #11 | broken | 14.3N | broken | - |
| Sanderlon | broken | 15.0N | broken | - |
| Zylone #0.8 | 0.14% | 26.5N | 12.3N | about 0.17 |
| Vectran #0.8 | 0.64% | 35.0N | 30.3N | about 0.17 |
| Technora 200d | 0.2% | 41.6N | 35.8N | about 0.20 |

It can be seen from this Table 1 that Technora is best in both shrinkage percentage and tensile strength, and especially, the tensile strength of Technora was hardly deteriorated even if Technora was exposed to an atmosphere of high temperature and high pressure.

Incidentally, Table 1 also shows that, in addition to aramid fiber, other kinds of fibers, such as polyacrylate fiber (tradename: Vectoran) and poly-p-phenylenebenzobisoxazole fiber (tradename: Zylone) also exhibited more or less excellent characteristics and hence are useful in the present invention.

Next, various examples of the present invention will be explained as follows.

### Example 1

This example illustrates one embodiment where the present invention was applied to the flexible tube of the endoscope.

FIG. 2 shows the flexible tube constituting an insertion part 2, wherein the optical fiber bundle, etc. which is inserted therein is omitted. The innermost layer of the flexible tube 10 is constituted of a helical tube 11 formed by helically winding a strip member so as to provide a tubular structure having a fixed diameter. The outer peripheral surface of the helical tube 11 is covered by a 2-ply layer of a net tube (braid) 12 which is formed by knitting yarn to tubular structure.

The outer peripheral surface of the net tube 12 is covered by an outer skin member 13 formed of a polyester tube. Alternatively, this outer skin member 13 may be formed by way of extrusion molding, dipping, etc. Incidentally, reference numeral 14 denotes a fluoroscopic indicator consisting of an X-ray impermeable member such as a thin lead plate for example.

In the flexible tube of the endoscope according to this example, the net tube 12 is formed using yarn of aramid fiber as a raw material. Therefore, the net tube 12 is excellent in flexibility and in mechanical strength, and is free from deterioration of the mechanical strength thereof even if the flexible tube is subjected to autoclave sterilization (high-pressure/high-temperature steam sterilization). Furthermore, this flexible tube 10 is very light in weight as compared with a net tube made of stainless steel. Incidentally, according to this example, although the net tube 12 is formed of a 2-ply structure, the net tube 12 may be formed of a single-ply structure.

In this example, the net tube 12 was formed using, as aramid fiber, copolypara-phenylene-3,4'-oxydiphenylene terephthalamide (tradename: Technora) and the endoscope was constructed using this net tube 12. Even when this endoscope was subjected to autoclave sterilization so as to expose this endoscope to a water vapor atmosphere of 135°C and 2 atm. for 5 minutes, it was possible prevent the yarn constituting the net tube 12 from being damaged through the rusting or disintegration thereof.

### Example 2

This example illustrates one embodiment where the present invention was applied to the fixing of the optical fiber bundle housed in the insertion part of endoscope.

The light guide and image guide housed inside the insertion part 2 shown in FIG. 2 are formed of an optical fiber bundle comprising a large number of fine optical fibers which are bundled en bloc. Since individual optical fiber constituting the optical fiber bundle is very fragile, the optical fiber bundle is inserted in a protective member in order to protect each of these optical fibers, thus forming, for example, an image guide assembly as a whole.

FIG. 3 shows an overall external view of one example of this image guide assembly. In FIG. 3, the optical fiber bundle 21 is inserted into and covered by a thin wall protective tube 22, thus forming an image guide main body 23. The distal end portion of the image guide main body 23 is fixed by way of the engagement of thin wall protective tube 22 with a mouthpiece 24. Yarn made of aramid fiber is wound around the outer peripheral surface of the thin wall protective tube 22 being engaged with the mouthpiece 24, thus forming a winding portion 25.

On the surface of the winding portion 25 wound in this manner around the outer peripheral surface of the thin wall protective tube 22, there is coated an adhesive.

In this example, the winding portion 25 was formed using, as aramid fiber, copolypara-phenylene-3,4'-oxydiphenylene terephthalamide (tradename: Technora), thereby fixing the light guide and the image guide in the insertion part 2. Even when the endoscope constructed in this manner was subjected to autoclave sterilization so as to expose this endoscope to a water vapor atmosphere of 135°C and 2 atm. for 5 minutes, it was possible to prevent the yarn constituting the winding portion 25 from being disintegrated and unfastened from the thin wall protective tube 22, thereby making it possible to prevent the optical fiber bundle 21 from being damaged.

### Example 3

This example illustrates one embodiment where the present invention was applied to the fixing of the channels housed in the insertion part of endoscope.

FIG. 4 is a cross-sectional view illustrating a secured portion formed between a channel pipe and a channel tube.

In FIG. 4, the connection between the channel pipe and the channel tube can be performed as follows.

First of all, a channel pipe 33 is inserted into the passageway 32 of the distal main body 31 of the insertion part 2. Then, a core bar 34 is inserted into the passageway 32 and into the channel pipe 33. This core bar 34 is constituted of a grip portion 35 and a cylindrical portion 36. Thereafter, a channel tube 37 covers on the outer peripheral surface of the cylindrical portion 36 of the core bar 34 which is protruded from the interior of the channel pipe 33. In this case, the proximal end portion of the channel tube 37 covers on the channel pipe 33.

Then, by way of manipulation by hand 39 for example, yarn 38 made of aramid fiber is wound several times around an outer peripheral portion of the channel tube 37 which is superimposed with the channel pipe 33, thereby forming a winding portion, on which an adhesive is subsequently coated to fix the winding portion.

The connection and fixing between the channel tube 37 and the channel pipe 33 explained above can be applied to various kinds of channels, such as a treatment tool channel, an air supply channel, a water supply channel, etc.

In this example, the connection and fixing between the channel tube 37 and the channel pipe 33 was performed using, as aramid fiber, copolypara-phenylene-3,4'-oxydiphenylene terephthalamide (tradename: Technora), and winding several times a fine aramid thread 38 around an outer peripheral portion of the channel tube 37 which was superimposed with the channel pipe 33. Even when the endoscope constructed in this manner was subjected to autoclave sterilization so as to expose this endoscope to a water vapor atmosphere of 135°C and 2 atm. for 5 minutes, it was possible to prevent the yarn 38 from being disintegrated, and the winding portion was prevented from being unfastened from the channel tube 37, thereby making it possible to prevent the channel tube 37 from being disengaged from the channel pipe 33.

The present invention would not be limited to the aforementioned examples, but can be variously modified without departing from the spirit of the present invention.

## Claims

1. An endoscope comprising:
an operation part (1); and
an intracavital insertion part (2) attached to the operation part (1) and formed of a flexible tube (10);
**characterized in that** the flexible tube (10) comprises a component member formed of yarn containing aramid fiber.

2. The endoscope according to claim 1, **characterized in that** the component member is a net tube (12) formed by knitting yarn containing aramid fiber into a tubular configuration.

3. The endoscope according to claim 1, **characterized in that** the flexible tube (10) comprises a helical tube (11) formed of a strip member which is helically wound to form a tubular configuration, a net tube (12) covering an outer peripheral surface of the helical tube (11), and an outer skin member (13) covering the outer peripheral surface of the net tube (12); and the component member is a net tube (12) formed by knitting yarn containing aramid fiber into a tubular configuration.

4. The endoscope according to claim 1, **characterized in that** the net tube (12) is fixed, through adhesion, to the outer skin member (13).

5. The endoscope according to claim 1, **characterized in that** the aramid fiber is copolyparaphenylene-3,4'-oxydiphenylene-terephthalamide obtained by copolymerizing terephthalic chloride and paraphenylene diamine using diamine having ether linkage as a third component.

6. The endoscope according to claim 1, **characterized in that** 50 mol% of said diamine is constituted of 3,4'-diaminodiphenyl ether, and said aramid fiber is a high-orientation fiber obtained by dry/wet spinning copolyparaphenylene-3,4'-oxydiphenylene-terephthalamide and then, by ultra-orientating the resultant fiber.

7. An endoscope comprising:
an operation part (1); and
an intracavital insertion part (2) attached to the operation part (1) and formed of a flexible tube (10);
**characterized in** the an internal member contained in the flexible tube (10) is secured to the flexible tube (10) by means of yarn containing aramid fiber.

8. The endoscope according to claim 7, **characterized in that** the internal member contained in the flexible tube (10) is an optical fiber bundle (21) comprising a large number of optical fibers and the outer peripheral surface of the optical fiber bundle (21) is covered with the thin tube (22), and an end portion of the optical fiber bundle (21) which is covered by the thin tube (22) is fixed by winding yarn formed of aramid fiber.

9. The endoscope according to claim 8, **characterized in that** a surface of the wound and fixed portion is coated with an adhesive.

10. The endoscope according to claim 7, **characterized in that** the internal member contained in the flexible tube (10) is at least one channel tube, **characterized in that** one end of the channel tube (37) covers an outer peripheral surface of the channel pipe (33) attached to a distal end portion of the intracavital insertion part (2), and yarn formed of aramid fiber is wound around the outer peripheral surface of the channel tube (37) overlapping the channel pipe (33).

11. The endoscope according to claim 10, **characterized in that** a surface of the wound and fixed portion is coated with an adhesive.

12. The endoscope according to claim 7, **characterized in that** the aramid fiber is copolyparaphenylene-3,4'-oxydiphenylene-terephthalamide obtained by copolymerizing terephthalic chloride and paraphenylene diamine using diamine having ether linkage as a third component.

13. The endoscope according to claim 7, **characterized in that** 50 mol% of said diamine is constituted of 3,4'-diaminodiphenyl ether, and said aramid fiber is a high-orientation fiber obtained by dry/wet spinning copolyparaphenylene-3,4'-oxydiphenylene-terephthalamide and then, by ultra-orientating the resultant fiber.

14. The endoscope according to claim 7, **characterized in that** the optical fiber bundle (21) is a light guide or an image guide (23).

15. The endoscope according to claim 10, **characterized in that** the channel tube (37) and the channel pipe (33) are selected from the group consisting of a treatment tool channel, an air supply channel, and a water supply channel.
